(19) Europäisches Patentamt
European Patent Office
Office européen des brevets

(11) **EP 4 503 043 A1**

(12) **EUROPEAN PATENT APPLICATION**

(43) Date of publication:
**05.02.2025 Bulletin 2025/06**

(21) Application number: **23189591.3**

(22) Date of filing: **03.08.2023**

(51) International Patent Classification (IPC):
**G16C 20/70** (2019.01)    **G16C 20/30** (2019.01)
**G16C 20/50** (2019.01)    **G06N 3/045** (2023.01)
**G06N 3/08** (2023.01)

(52) Cooperative Patent Classification (CPC):
**G16C 20/70; G06N 3/045; G16C 20/30;**
**G16C 20/50;** G06N 3/08

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Helmholtz-Zentrum für**
**Infektionsforschung GmbH**
**38124 Braunschweig (DE)**

(72) Inventors:
• **Asgari, Ehsannedin**
 **38124 Braunschweig (DE)**
• **McHardy, Alice**
 **38124 Braunschweig (DE)**
• **Kallergis, Georgios**
 **38124 Braunschweig (DE)**

(74) Representative: **Patentanwälte Bressel und**
**Partner mbB**
**Potsdamer Platz 10**
**10785 Berlin (DE)**

(54) **A COMPUTER-IMPLEMENTED METHOD FOR TRAINING A MODEL FOR USE IN ESTIMATION OF AT LEAST ONE PROPERTY OF A CHEMICAL COMPOUND AND/OR FOR GENERATING OR OPTIMIZING CHEMICAL COMPOUNDS**

(57) The invention relates to a computer-implemented method for training a model (13) for use in estimation of at least one property (16) of a chemical compound and/or for generating or optimizing chemical compounds, the model comprising a language model (13-1), the method comprising the steps of:
a) Training the language model (13-1) using a large number of chemical compounds in a string representation (10) as training data,
b) Performing domain adaption of the trained language model (13-1) of step a) for a target domain using target domain-specific training data of the chemical compounds,
c) Training the domain-adapted trained model (13) of step b) by supervised training for a specific molecular or other property prediction task or a specific chemical compound generation or optimization task.

The invention further relates to a computer program, a data-processing system, uses of the trained model (13).

Fig. 4

**Description**

The invention relates to a computer-implemented method for training a model for use in estimation of at least one property of a chemical compound and/or for generating or optimizing chemical compounds, a computer program, a data-processing system, and uses of the trained model.

Drug development requires a huge amount of money and time. It is estimated that 1.8 billion dollars investment is required and almost 20 years to develop a single drug [1], with an overall failure rate of about 96 % [2]. In addition, medicinal chemists must discover the appropriate chemical structure in a vast chemical space; the number of drug-like compounds is $10^{60}$. Hence, they synthesize hundreds of compounds, most of which will fail in the very early stages until they find the one that may succeed in the clinical trials. That leads to a long and expensive procedure that certainly needs computational methods' assistance to cut down the costs and speed up drug compound discovery and assessment by applying these models in key stages or tasks of this process.

In silico approaches, such as machine learning, in particular deep learning algorithms, have the potential to accurately capture the relationships and properties of molecules [3, 4], and thus support and facilitate drug development by suggesting potential drug candidates.

Machine learning models have already been deployed and trained on chemical descriptors, such as fingerprints, which take into account the chemical characteristics of compounds [5, 6]. Sparse vectors and bit-collision problems were some of the fingerprints' drawbacks, though. The limitations of fingerprints led to representing chemical compounds either as natural graphs or as string representations that encode all the necessary chemical information. Graphs are used as input to Graph Neural Networks (GNNs) [7-9], which dominate the field nowadays. There is a plethora of these models, with different variations that all share the aggregation of information and the update across the graph according to the type of their architecture [10-13]. Treating molecules as graphs makes them topologically aware, among other advantages. However, they also have certain weak points in these architectures, such as their input does not include information such as the branches or chirality that string representations do and they are restricted to depth and if increasing, can lead to oversmoothing.

Language methodologies arise as potential solutions. The prevailed string representation of compounds is SMILES, which stands for Simplified Molecular-Input Line Entry System [14]. It is the result of a depth-first preorder spanning tree traversal of the molecular graph. Chomsky's definition of language allows to move beyond the usual borders of natural language and to reconsider what can be perceived as a language [15]. Proteins have already been treated using this perspective, as their finite length and the finite number of amino acids resemble a language, "a protein language" [16-19]. Similar to proteins, SMILES meets the language definition and can be processed as a language task [20]. As a result, language processing methodology and techniques have been utilized starting with Word2Vec approaches [21-23], and later, with Recurrent Neural Networks (RNNs) [20, 24]. The recent rise of Transformers revealed a new potential in the language processing field [25]. Their novel architecture allows them to take advantage of a large amount of data and achieve state-of-the-art results. Recent work in this field has revealed that Transformers can make an impact in this field [26, 27].

Transformers aim to take advantage of transfer learning, a widely used concept in machine learning. Briefly, a model is trained on a related or more general problem with abundant training data, and then adapted or used for a target task with limited data, resulting in improved performance and accelerated convergence. Although transfer learning was primarily developed for supervised machine learning tasks, its application has expanded to self-supervised tasks [28-30] and allows to pre-train a model on millions of molecules from public databases (e.g. ZINC, PubChem, DrugBank).

The invention is based on the technical problem of developing an enhanced solution for training a model for use in estimation of at least one property of a chemical compound and/or for generating or optimizing chemical compounds of given properties of interest. In particular, the prediction quality and/or generation or optimization quality should be enhanced.

According to the invention, the technical problem is solved by the subject matter of the independent claims. Advantageous embodiments of the invention emerge from the dependent claims.

In particular, a computer-implemented method is provided for training a model for use in estimation of at least one property of a chemical compound and/or for generating or optimizing chemical compounds, the model comprising a language model, the method comprising the steps of:

a) Training the language model using a large number of chemical compounds in a string representation as training data,
b) Performing domain adaption of the trained language model of step a) for a target domain using target domain-specific training data of the chemical compounds,
c) Training the domain-adapted trained model of step b) by supervised training for a specific molecular or other property prediction task and/or a specific chemical compound generation or optimization task.

In a second aspect of the invention, in particular a computer program is proposed having instructions which when executed by a computing device or system cause the computing device or system to perform the method according to any one of the embodiments disclosed herein. The computer program may be part of a computer program product and may be stored on a non-transitory computer-readable storage medium.

Further, in a third aspect of the invention, in particular a device is proposed comprising means for carrying out the method according to any one of the embodiments disclosed herein. The means may comprise at least one processing device and at least one memory. The means may further comprise a communication bus, and an input/output interface for receiving and/or transmitting data and/or signals.

In a fourth aspect of the invention, in particular, the invention provides a use of the model trained according to any one of the embodiments described in this disclosure to predict at least one molecular or other property and/or a drug-target interaction and/or a drug potency and/or a binding to proteins of a chemical compound.

In a fifth aspect of the invention, in particular, the invention provides a use of the model trained according to any one of the embodiments described in this disclosure to generate or optimize one or more chemical compounds with at least one specific target property. The term "generation" is used in this disclosure with the meaning of suggesting or determining chemical compounds, in particular, suggesting or determining (tokenized) string representations of the chemical compounds.

In a sixth aspect of the invention, in particular, the invention provides a trained (machine learning) model obtainable by a training method according to any of the embodiments described in this disclosure.

Further the invention provides, in particular, a weight configuration of a trained (machine learning) model obtainable by a training method according to any of the embodiments described in this disclosure.

The method, the computer program, the data-processing system, the uses, and the trained (machine learning) model allow enhancing the prediction quality of the estimation of at least one property of a chemical compound and/or enhancing the quality of the generation or optimization of chemical compounds. One of the key ideas of the invention is to use domain adaptation of the generally trained language model to specialize the language model in a specific target domain using target domain-specific training data of the chemical compounds. The target domain of this domain adaptation step corresponds directly to the final prediction or generation/optimization task the model is trained for in step c). In other words, the method, in particular, comprises three main steps or stages: (i) pre-training the language model using a large database (step a), (ii) domain adaptation to the target domain (step b), and (iii) supervised fine-tuning of the model to a specific task (step c).

In particular, the final model comprises a language model part equipped with a prediction part for property prediction and/or a generation/optimization part for producing compounds with a property of interest.

The language model is fed with tokenized strings resembling the chemical compounds. As a framework for the string representation of the chemical compounds, for example, SMILES (Simplified Molecular-Input Line Entry System), InChi or SMARTS may be used. Preferably, SMILES is used. For the tokenization, Byte Pair Encoding (BPE) may be used. In particular, chemical-based as well as computational-based approaches may be used. Preferably, a computational-based approach is used. Each string representation is considered equivalent to a sentence consisting of many tokens. In particular, Byte-Level Byte Pair Encoding (BBPE) is used [39]. The BPE algorithm splits the sequence into the most frequent subsequences or bytes. Hence, it can find potential substructures, which may then be considered as tokens. Another advantage is the user-defined vocabulary size which is equivalent to the total number of tokens at the end of the procedure. Hence, the bigger it is, the more pairs will be included leading to different tokenization. In particular, the vocabulary size is chosen to be ~10000. In addition to the earlier mentioned benefits, the Byte-level approach is more compact, utilizing only bytes. In particular, to learn the underlying sequence of bytes, the BBPE tokenizer is trained in a large corpus of SMILES (or another string representation), such as the ZINC, PubChem and/or DrugBank database. This tokenizer can be used in different applications or datasets.

Language models are chosen based on the specific task at hand. Autoregressive models such as GPT are commonly employed for generation tasks. On the other hand, transformer models like RoBERTa find utility in classification and chemical labeling applications. Additionally, there are other models based on Bidirectional Encoder Representations from Transformers (BERT) architecture that serve as alternatives to RoBERTa, including DistillBERT, ELECTRA, and RoFormer, which incorporates rotary position embeddings.

Another option is to use encoder-decoder architectures like the Bidirectional and AutoRegressive Transformer (BART) model. BART has been successfully applied in the ChemFormer approach, proving useful for multiple chemical tasks, such as reaction synthesis prediction and molecular property prediction.

The transformer language model is an encoder architecture [25] using multi-head attention. At the core of multi-head attention lies the concept of self-attention, which focuses on generating improved representations of the sequence elements (tokens) by considering their interactions with neighboring elements. This self-attention mechanism is utilized within multi-head attention to enable the model to attend to multiple views of the sequence interactions simultaneously, resulting in more expressive and informative representations.

In a broad definition of attention, each token of the sequence is associated with two real-valued vector representations: (i) a key vector (k) from the input embedding space and (ii) a value vector (v) from the output embedding space. These vectors can be either randomly initialized or pre-trained. The query vector (q) represents the sequence element for which one wants to obtain a new representation and must belong to the same space as the key vectors. To calculate a new representation for the entire sequence, the key, query, and value vectors are organized into matrices $K$, $Q$, and $V$, respectively. In self-attention, the rows of matrices $Q$ and $K$ are even identical.

The attention layer output is obtained through these steps: (i) The pair-wise interaction of sequence elements using matrix multiplication of Q and $K^T$ are measured. (ii) SoftMax layer is applied to create a probability distribution of these interactions for each element across the entire sequence. The temperature of softmax is controlled by $d_k$ (vector k's dimension). (iii) The output of each element is computed as a weighted sum of v vectors, where the weights come from the softmax.

$$Attention(Q, K, V) = softmax\left(\frac{QK^t}{\sqrt{d_k}}\right) \tag{1}$$

Since multiple views of similarities exist between sequence elements, instead of performing a single attention mechanism, a multi-head one maybe used:

$$MultiHead(Q, K, V) = Concat(head^1, ..., head^h)W^0 \tag{2}$$

where each $head^i$ is equal to

$$head^i = Attention(QW_i^Q, KW_i^K, VW_i^V) \tag{3}$$

and $W_i$ is the weight matrix. Another key element is positional embedding to provide information about the position of tokens in the sequence and counteract the absence of recurrent or convolutional elements.

$$PE_{(pos,2i)} = sin\left(\frac{pos}{10000^{\frac{2i}{d_{model}}}}\right) \tag{4}$$

$$PE_{(pos,2i+1)} = cos\left(\frac{pos}{10000^{\frac{2i}{d_{model}}}}\right) \tag{5}$$

The mentioned architecture is used to predict randomly masked elements in the sequence, helping the model understand sequence structure and relationships between elements. Models with only the encoder can be used for molecular property prediction, while models with both encoder and decoder or just a decoder can be employed in drug generation.

The language model takes input data as a sequence of token representations. It produces a real-valued vector representation (embedding) that captures abstract features in a semantic space, bringing similar elements closer together. The vector representation doesn't directly correspond to real input features.

The workflows of the model may be implemented using HuggingFace [40] and pytorch [41].

Training the language model in step a) is performed, in particular, using unlabeled data. For example, the ZINC database, a public collection of more than 900 million compounds [37], may be used for this purpose. Not all of the compounds in the database may be used for the training, but only a pre-selected number of the chemical compounds. For chemical compounds (molecules) in the database, a string representation (e.g. using SMILES) is retrieved and then used for training the language model in step a). The string representation is tokenized for this purpose. In particular, masked language modeling (MLM, [28]) may be used to train the language model. This technique masks random tokens of the input sequence and trains the language model by predicting the masked token based on the surrounding ones. In other words, during step a), the language model learns the chemical and structural information of the chemical compounds (molecules) used during training developing an understanding of the chemical language.

In step b), the pre-trained language model gets domain-adapted to a target domain. In principle, the training is similar to that in step a), just for that purpose, training data coming from the target domain is used. The training data does not need to include labels, different from the requirements of training data utilized in step c). The goal is to train the model with data related to the final task so that it can focus on domain representations and leverage the chemical knowledge for this task. The training data for step b) may be the same training data used in step c) while disregarding/not using the corresponding labels. The result of step b) is a domain-adapted pre-trained language model.

In step c), the domain adapted pre-trained model of step b) is trained by supervised training for a specific molecular or other property predication task. For the training, labeled training data is used. The training data may comprise data pairs, each comprising a chemical compound (in string representation) and corresponding labeling data (i.e. information on at least one molecular or other property). To prevent overfitting, early stopping may be deployed apart from techniques in model development like L2 regularization. In particular, a neural network (classification/regression head) is attached on top to make the predictions in step c). The difference between the classification and regression head is the final layer, in which either n neurons for n classes in a classification problem or one for a regression task are used. As described above, the language model provides a vector representation (embedding) as output. In the case of property prediction, this vector representation is fed, in particular, to the classification/regression head, which connects the vector representation with the output(s) corresponding to the at least one (predicted) property. In particular, these further layers are trained in a supervised way during step c) to map the vector representation to the property output(s).

In the case of a generation or optimization task, a model with encoder/decoder architecture has to be used instead of the encoder model for molecular property prediction. However, the training steps remain the same. Both encoder and the decoder are initialized with the 2 phases of general purpose and domain specific language modeling. The encoder encodes the properties of interest and/or the initial sequence and the decoder produces a probability distribution over the SMILES (or another string representation) vocabulary for the i-th token of the generated sequence. The properties of interest can be encoded to the same space as SMILES embedding. Then, a token is selected using an algorithm, such as beam search, which selects the tokens with the highest probability to fit in the generated sequence given the previously selected tokens. Loss is an important measure that indicates how well the model is trained. Preferably, in categorical prediction (sequence classification/generation) settings, cross entropy loss is used; however, other functions could be utilized successfully or even a combination of different functions. In this way, valid SMILES sequences are created. For the training in step c), in particular pairs of SMILES sequences are used as training data. The input sequence to train the model and give the prompt for the generation can vary. These inputs can be provided as SMILES representations, which can be modified and/or modified SMILES sequences by adding extra tokens to guide the generation. For example, training data for use in step c) may comprise data pairs of a SMILES representation of a chemical compound with a certain property as input paired with a SMILES representation of a similar chemical compound, but with a different value for this property (e.g. a different lipophilicity), as output. Using an extra token in the input sequence the change (increase/decrease) of this property and the direction of change may be indicated. This allows to control the direction of property change when later applying the trained model. In other words, starting with a chemical compound the extra tokens can be set in the SMILES representation to indicate the desired direction of property change. For instance, in previous works tokens have been used for each property, to instruct the model how SMILES sequences should be changed to alter the property value, e.g. increase or decrease lipophilicity.

Moreover, target sequences (for a target molecule that the generated molecule should interact with in a certain way, e.g. a protein) can be used to generate compounds that, for example, inhibit these targets. For this purpose, training data is used comprising pairs of the target sequence as input sequence paired with chemical compounds, which are known to inhibit the target (e.g. a protein), represented in the target sequence as output sequence. Similar to a machine translation task in language processing, where a sentence in English can be translated into a sentence in French, drug generation can be viewed as a machine translation problem. By providing a target sequence, one can obtain a SMILES sequence as the output. To achieve that, the model is trained on pairs of targets and SMILES sequences and learns the characteristics that the chemical compound needs to have to interact in the desired way with that target. Thus, the generated SMILES will interact with the input target in the desired way.

Controllable generation can be achieved in many ways. Those include the addition of attribute embeddings to affect the generation of the sequence, adjusting the score function to include and/or quantify specific characteristics or even transformer models like CTRL.

Further, reinforcement learning may be used to control the SMILES generation or optimization. Reinforcement learning uses a reward model for desired generated or optimized sequences. This model will be based on an evaluation metric regarding a given goal, such as generating a compound that has specific molecular weight and aims to maximize that award. After producing multiple sequences, the model is rewarded for each sequence with desired properties. There are many techniques on how to affect the generation using reinforcement learning, one of which is policy gradients that uses gradient ascent to optimize the policy parameters in order to find the best policy. In the last step, a combination of reinforcement learning's reward and the model's loss regarding the generation sequence may be utilized to fine-tune it.

The following table lists examples for molecular properties that may be predicted with the model after using the method described in this disclosure.

| Molecular property of the chemical compound |
|---|
| Drug-target interaction |
| Drug potency |
| Binding to proteins |
| Toxicity |
| Lipophilicity |
| Free solvation energy |
| Permeability |
| Aqueous solubility |
| Stability |
| Bioavailability |
| Size / molecular weight |

In one embodiment, additional training data is generated using data augmentation and is used at least in step b). This allows to support the domain adaptation task, i.e. the specialization and the performance of the language model for the domain corresponding to the final specific task may be enhanced.

In one embodiment, for augmenting the training data, the string representations of chemical compounds are reordered leading to multiple string representations of a molecule. In particular, SMILES enumeration [45] may be used to augment the training data. The number of augmentations (e.g. 5, 10, 20, 40, 60, 80, 100, ...) may be part of an additional optimization step in order to further enhance the performance of the model.

In one embodiment, the string representation of the chemical compounds is tokenized by using Byte Pair Encoding (BPE).

In one embodiment, only a prediction part of the model is trained during step c) while parameters of the language model are kept constant during step c). This can save computation power and time, mainly on large datasets with thousands of compounds.

In an alternative embodiment, the language model and a prediction part of the model are trained during step c). This allows to further increase the performance of the model since the language model may also be adapted (fine-tuned) during the supervised training in step c).

In one embodiment, hyperparameters of the model and/or an embeddings' type of the language model and/or an augmentation size are optimized using the training data of step c) and/or at least one benchmark dataset. This allows to further increase the performance of the model. Hyperparameters are, for example, a number of hidden layers used in the language model and/or the number of attention heads used and/or hidden units per layer and/or activation functions and/or, training task, and/or embeddings size. The embeddings type resembles a way to combine the high-dimensional vector representation of the input generated by the hidden layers of the language model. The embeddings type resembles how the vector representation that is provided as output (of the encoder) of the language model is created or assembled. For example, different outputs of different hidden layers may be considered when constructing the vector representation. The model may be evaluated with part of the data used as validation data in step c). For this purpose, part of the labeled training data is not used for training, but for evaluation of the optimization steps. For example, models with different sets of hyperparameters, e.g. different combinations of hidden layers and/or attention heads, may be trained in the way described in this disclosure and then, after complete training, evaluated using a specific metric, such as, for example, the f1-metric and/or the AUC-metric, on the validation set. The hyperparameters resulting in the highest values for these metric(s) may be selected and used for the application of the trained model.

Further, in particular a computer-implemented method for controllable generation or optimization of drugs is also provided, wherein a model trained according to any of the embodiments disclosed herein is used within a learning framework to controllably generate or optimize one or more chemical compounds. The method allows to explore and find or optimize different chemical compounds with the purpose of finding or optimizing at least one specific property.

In one embodiment, the learning framework implements reinforcement learning.

In addition, in particular a method for predicting at least one property of one or more chemical compounds and/or for generating or optimizing at least one chemical compound is provided, wherein the method comprises the computer-implemented method according to any of the embodiments disclosed herein, and wherein the method further comprises selecting a group of chemical compounds with at least one given target property and/or determining at least one molecular or other property of the group and using at least part of the group for domain adaption in step b) and/or supervised training in step c). This allows to define the target domain as well as to provide the training data for step b) and/or step c). The at least one molecular or other property is, in particular, experimentally determined.

Further, in particular a method for generating or optimizing at least one chemical compound is provided, wherein the method comprises the computer-implemented method according to any of the embodiments disclosed herein, and wherein the method further comprises selecting a group of pairs of chemical compounds, wherein the chemical compounds in each pair differ in a value of at least one chemical or other property, and using at least part of the group for supervised training in step c). This allows to specifically train the model for the generation or optimization task.

Also, in particular a method for generating or optimizing at least one chemical compound is provided, wherein the method comprises the computer-implemented method according to any of the embodiments disclosed herein, and wherein the method further comprises selecting a group of pairs of chemical compounds, wherein the pairs comprise a target compound paired with a chemical compound, wherein the pairing is such that the target compound and the chemical compound show a desired interaction, and using at least part of the group for supervised training in step c). This allows to train the model for the generation or optimization task, in particular for generating or optimizing chemical compounds which show a specific interaction with the target compound.

The invention is explained in greater detail below on the basis of preferred exemplary embodiments with reference to the drawings. In the drawings:

Fig.1    shows a schematic of an embodiment of the data-processing system used for performing the method;

Fig.2    shows a schematic diagram to illustrate an embodiment of the method;

Fig.3    shows a schematic illustrating an example of the data workflow within the method;

Fig.4    shows a schematic to illustrate the three stages of the training according to the method;

Fig.5    shows a schematic diagram illustrating the use of the model during a generation or optimization of a chemical compound;

Fig. 6    shows a schematic diagram illustrating an embodiment of a computer-implemented method for controllable generation or optimization of drugs.

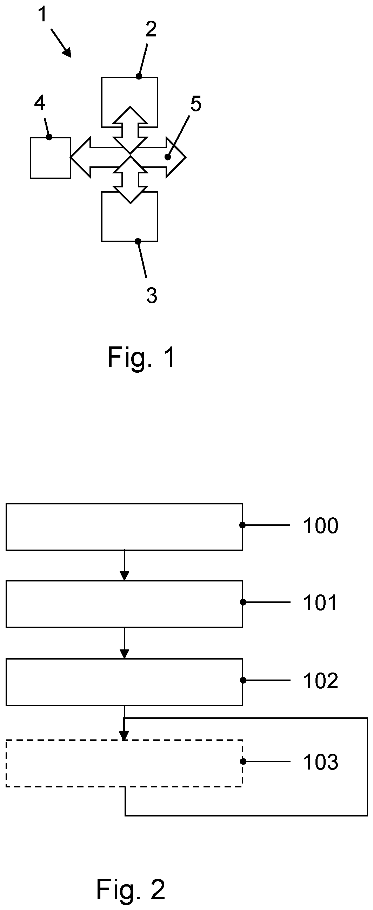

Fig. 1 shows a schematic view of an embodiment of the data-processing system 1 used for performing the method described in this disclosure. The data-processing system 1 comprises at least one processor 2, at least one memory 3, an input/output interface 4, and a communication bus 5 connecting the processor 2, the memory 3 and the interface 4. The data-processing system 1 may be a computer or a cloud computing platform.

Fig. 2 shows a schematic diagram illustrating an embodiment of the method for training a model for use in estimation of at least one property of a chemical compound and/or for generating or optimizing chemical compounds. The method may be performed on the data-processing system1 shown in fig.1.

In step 100, a model is provided on a data-processing system. The model comprises of a language model.

In step 101, the language model is trained using a large number of chemical compounds in a string representation as training data. The training is performed with unlabeled data. The large number of chemical compounds may be derived from the ZINC, PubChem and/or DrugBank databases. The chemical compounds are represented as character strings, for example, using the SMILES, InChi or SMARTS representation. The character strings are tokenized using a tokenizer; for example, tokenization is done by Byte Pair Encoding (BPE), in particular Byte-Level Byte Pair Encoding (BBPE). Training is carried out by using masked language modelling (MLM) [28], such that the language model learns the semantic and syntax information contained in the number of chemical compounds.

In step 101, a domain adaptation is performed on the language model trained in step 101. In particular, a target domain is selected by selecting specific training data of the chemical compounds corresponding to that target domain. The target domain is, in particular, the target domain that corresponds to the final prediction or generation or optimization task. Optionally, the training data used in step 101 may be augmented using a particular augmentation size (number of additional data items per original data item), in particular by using enumeration techniques, for example, SMILES enumeration [45]. In particular, for augmenting the training data, part (e.g. atoms) of the string representation of the chemical compounds may be reordered leading to multiple representations of a molecule, thus increasing the number of training data.

In step 102, the domain-adapted trained model resulting from step 101 is trained by supervised training for a specific molecular or other property predication task and/or a specific chemical compound generation or optimization task. For the supervised training, labeled training data is provided and/or created, in particular data pairs, comprising of a chemical compound as input data paired with property data (e.g. at least one molecular or other property) for prediction.

For generation or optimization, labeled training data is provided and/or created, in particular data pairs, comprising of a chemical compound as input paired with chemical compounds with at least one altered property for generation/optimization as output data.

During step 102, only a prediction part of the model may be trained while parameters of the language model are kept constant. Alternatively, the prediction part of the model and the language model may be trained during step 102.

Optionally, as part of an additional step 103, hyperparameters of the model and/or an embeddings' type of the language model and/or an augmentation size may be optimized using the training data of step 102 and/or at least one benchmark dataset. The benchmark datasets may be BACE and/or BBBP and/or ClinTox from the MoleculeNet [38], which are related to physicochemical properties of molecules. For the optimization, the benchmark datasets are used to test the performance of the model. In particular, the quality of prediction of the at least one molecular or other property, expressed for example as "area under the ROC curve" (AUC) value, is used as optimization criterion.

The method may be used, in particular, for zero- or few-shot learning. For zero-shot learning, no (additional) training is performed before predicting at least one molecular or other property of a chemical compound. For few-shot learning, only a small number of labeled training data is necessary for the supervised training step. These ways allow the model to predict at least one molecular or other property of the chemical compounds, even if no or few labeled training data are available.

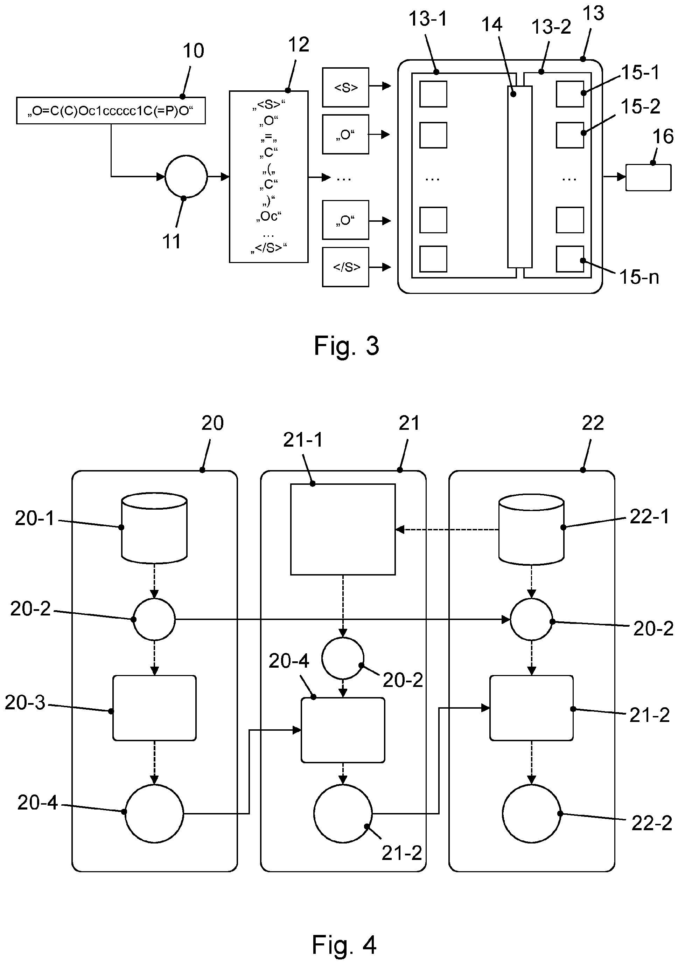

Fig. 3 shows a schematic illustrating an example of the data workflow within the method for a prediction task. The chemical compounds (molecules) are represented in a string representation 10 (an exemplary SMILES representation is depicted). The string representation 10 of the chemical compound is then tokenized by a tokenizer 11, in which the string is split into a sequence 12 of tokens. Special tokens (e.g. <s>, <\s>) are added to the sequence 12. The individual tokens of the sequence 12 are fed into the model 13. The model is, in particular, a neural network, in particular a deep neural network with a number of hidden layers. The model comprises a language model 13-1 and a prediction part 13-2. In particular, the language model 13-1 is of the Transformer type, comprising an encoder. The result from the language model 13-1, a vector representation 14 ("embedding") of the input of the language model 13-1, is fed to the prediction part 13-2 of the model 13. However, a strict separation of these parts 13-1, 13-2 is not necessary. The sum of weights from hidden layers of the model 13 is used to make the prediction. The outputs 15-x of the model 13, in particular the predication part 13-2 of the model 13, are corresponding to the molecular or other properties 16 that are learned during the training and that are predicted during application (inference) of the trained model13.

Fig. 4 shows a schematic to illustrate the three stages 20, 21, 22 (named steps a, b, and c in the general description and the claims) of the training according to the method. In the first stage 20, for example, a number of ten million chemical compounds is taken from the ZINC or another database 20-1, represented as strings, and tokenized by the BBPE tokenizer 20-2. The model, comprising the language model 20-3, in particular a Transformer model, is trained using the 10 million chemical compounds and masked language modeling (MLM), which results in a trained model 20-4.

In the second stage 21, the trained model 20-4 is domain-adapted to a target domain, using, in particular, augmented training data 21-1 from the target domain. The augmentation may be done by SMILES enumeration. The training of the second stage 21 is basically the same as in the first stage 20: the augmented training data 21-1 in string representation is tokenized using the same BBPE tokenizer 20-2, then the trained model 20-4 is domain-adapted by training it again with the augmented training data 21-1, resulting in a domain-adapted trained model 21-2.

In the third stage 22 of the training, the model 21-2 is trained for the specific prediction or generation or optimization task. This is done by supervised learning using labeled training data 22-1 within the target domain. The labeled training data 22-1 may come from a benchmark dataset or may be experimentally created. The labels contain information about the at least one molecular or other property. In case of a prediction task, the labels for the chemical compounds of the training data 22-1 are, in particular obtained by experimental tests and/or by simulation. In case of a generation or optimization task, the labels are in particular also string representations of chemical compounds. In particular, a number of ~1000 to ~3000 pairs are used as labeled training data 22-1. The labeled training data 22-1 may also be used as domain-specific training data 21-1 in the second training stage 21 as indicated by the dashed arrow. In this case, the labels are disregarded/not used during the unsupervised or self-supervised training in the second stage 21.

The supervised training in the third stage 22 results in a domain-adapted fine-tuned model 22-2, which may be used to predict at least one molecular or other property of a chemical compound which is input to the domain-adapted fine-tuned model 22-2 and/or to generate or optimize a chemical compound with given properties.

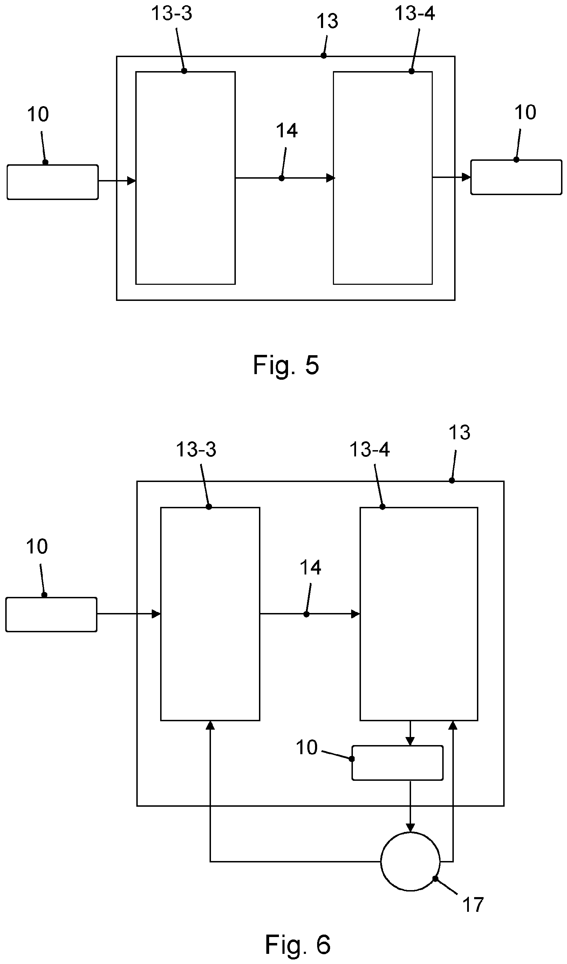

Fig. 5 shows a schematic diagram illustrating the use of the model during a generation or optimization of a chemical compound. The goal is to generate a chemical compound with a specific property or to optimize a specific property.

The model 13 for generation or optimization of at least one property is of the encoder/decoder type, i.e. it comprises an encoder 13-3 and a decoder 13-4. The encoder 13-2 encodes an input string representation 10 to a vector representation 14 ("embedding") and the decoder 13-4 decodes ("suggests") a string representation 10 based on the vector representation 14. The encoder 13-3 and decoder 13-4 are trained in a general way as described above (steps a) and b); fig. 1, steps 100 and 101; fig. 4, first stage 20 and second stage 21). The model input is a string representation 10 (e.g. a SMILES sequence), the model output is also a string representation 10 (e.g. a SMILES sequence).

For the specific generation or optimization task, supervised training is performed using pairs of (tokenized) string representations 10 as training data (= step c).

In one example, the pairs comprise as input a string representation 10 of a chemical compound and as output a string representation of a chemical compound with altered properties in comparison to the chemical compound of the input. Extra tokens in the string representation of the input may be used to indicate the property and the direction of property change from the input to the output chemical component. After training and during application the model can generate string representations 10 of chemical compounds with altered properties based on a string representation 10 of a chemical compound as input. Using the extra tokens allows to direct the change of the at least one property. This can, in particular, be used to optimize chemical compounds with regard to desired properties.

In another example, a target sequence of a target molecule (e.g. a protein) may be used to generate chemical compounds, which show a desired interaction with this target. In this case, for training, the pairs comprise as input a string representation 10 of the target sequence and as output string representations 10 of chemical compounds, which show the desired interaction with the target (e.g. inhibiting the target). After training and during application the model can generate string representations 10 of chemical compounds, which show the desired interaction with the target (e.g. inhibiting the target) based on a string representation 10 of a target.

Fig. 6 shows a schematic diagram illustrating an embodiment of a computer-implemented method for controllable generation or optimization of drugs. A model 13 trained according to any of the embodiments described in this disclosure is used within a learning framework to controllably generate or optimize one or more chemical compounds. In particular, the model 13 has been trained according to the examples provided with respect to fig. 5.

In particular, the learning framework implements reinforcement learning. For this purpose, a reinforcement agent 17 controls the generation or optimization. Reinforcement learning uses a reward model for desired generated sequences. It is based on an evaluation metric regarding a given goal, such as generating a compound that has a specific molecular weight and aims to maximize that award.

Starting with a string representation 10 of a chemical compound the model 13, in particular the decoder 13-4, produces multiple sequences as string representations 10. After producing the multiple sequences, the model 13 is rewarded for each sequence with desired properties. Based on the rewards, the next iteration is started, eventually leading to a string representation 10 of a chemical compound with the desired properties.

There are many techniques on how to affect the generation using reinforcement learning, one of which is policy gradients that uses gradient ascent to optimize the policy parameters in order to find the best policy. A combination of reinforcement learning's reward and the model's loss regarding the generation sequence may be utilized to fine-tune it, i.e. also the parameters of the model 13 may be adapted during the reinforcement learning.

Prediction quality

Regarding the prediction quality, the model created with the method described in this disclosure outperforms other methods in the field, making it advantageous for application.

Table 1 indicates the F1 scores for the model described in this disclosure and for other models for different benchmark datasets.

Table 1:F1 score values for various benchmark datasets and models

| Model | BBBP | BACE | ClinTox |
|---|---|---|---|
| MPNN | 0.794 | 0.741 | 0.754 |
| GAT | 0.7852 | 0.73 | 0.7 |
| GCNN | 0.7851 | 0.77 | 0.637 |
| This model optimized | 0.836 | 0.801 | 0.822 |

The BBBP dataset was split in a stratified way whereas the rest used scaffold using DeepChem's splitter [36]. They were split into 70% training, 10 % validation and 20% test sets. The model for training described in this disclosure ("this model") was compared to state-of-the-art models: Graph Convolution Neural Networks (GCNN), Graph Attention Transformers (GAT) and Message Passing Neural Networks (MPNN) whose implementations were found in DeepChem and were used with the default architecture they had. The hyperparameters were optimized using a grid search in the same hyperparameter space as in this model. DeepChem framework was used for that. For the optimized model ("This model, optimized"), optimization was done by using domain adaptation combined with data augmentation. In addition to that, different embeddings' types were tested in order to find the optimal one. For small datasets like BACE, instead of freezing the language model's layers, they were also fine-tuned as that boosts the performance. This, of course, cannot be done in every dataset due to memory issues but even for larger ones, it is possible to at least unfreeze some of the layers of the language model. This model yields better results compared to the other graph models (Table 1). It outperforms them for all datasets showing great performance and a significant improvement. All the optimizations in different parts of the models (augmentation size, embeddings' type as input to the classification head) play an important role.

Reference numeral list

| | |
|---|---|
| 1 | data-processing system |
| 2 | processor |
| 3 | memory |
| 4 | input/output interface |
| 5 | communication interface |
| 10 | string representation |
| 11 | tokenizer |
| 12 | sequence of tokens |
| 13 | model |
| 13-1 | language model |
| 13-2 | prediction part of model |
| 13-3 | encoder |
| 13-4 | decoder |
| 14 | vector representation ("embedding") |
| 15-x | outputs of model |
| 16 | molecular or other property/properties |
| 17 | reinforcement learning agent |
| 20 | first training stage |
| 20-1 | chemical compound database (e.g. ZINC) |
| 20-2 | (BBPE) tokenizer |
| 20-3 | language model |
| 20-4 | trained model |
| 21 | second training stage |
| 21-1 | augmented training data |
| 21-2 | domain-adapted trained model |
| 22 | third training stage |
| 22-1 | labeled training data |
| 22-1 | domain-adapted fine-tuned model |
| 100-104 | method steps |

Literature

[1] Paul, S.M., Mytelka, D.S., Dunwiddie, C.T., Persinger, C.C., Munos, B.H., Lindborg, S.R., Schacht, A.L.: How to improve RD productivity: The pharmaceutical industry's grand challenge 9(3), 203-214 (2010). https://doi.org/10.1038/nrd3078

[2] Hingorani, A.D., Kuan, V., Finan, C., Kruger, F.A., Gaulton, A., Chopade, S., Sofat, R., MacAllister, R.J., Overington, J.P., Hemingway, H., Denaxas, S., Prieto, D., Casas, J.P.: Improving the odds of drug development success through human genomics: modelling study. Scientific Reports 9(1), 18911 (2019). https://doi.org/10.1038/s41598-019-54849-w

[3] Ma, J., Sheridan, R.P., Liaw, A., Dahl, G.E., Svetnik, V.: Deep neural nets as a method for quantitative structure-activity relationships. Journal of Chemical Information and Modeling 55(2), 263-274 (2015). https://doi.org/10.1021/ci500747n

[4] Chen, H., Engkvist, O., Wang, Y., Olivecrona, M., Blaschke, T.: The rise of deep learning in drug discovery. Elsevier Ltd (2018). https://doi.org/10.1016/j.drudis.2018.01.039

[5] Zhang, Q.Y., Aires-de-Sousa, J.: Random forest prediction of mutagenicity from empirical physicochemical descriptors. Journal of Chemical Information and Modeling 47(1), 1-8 (2007). https://doi.org/10.1021/ci050520j

[6] Zernov, V.V., Balakin, K.V., Ivaschenko, A.A., Savchuk, N.P., Pletnev, I.V.: Drug Discovery Using Support Vector Machines. The Case Studies of Drug-likeness, Agrochemical-likeness, and Enzyme Inhibition Predictions. Journal of Chemical Information and Computer Sciences 43(6), 2048-2056 (2003). https://doi.org/10.1021/ci0340916

[7] D Duvenaud, D Maclaurin, J.A.-I.: Convolutional networks on graphs for learning molecular fingerprints. Adv Neural Inf Process Syst 2015, 2224-2232 (2015)

[8] Y, X., J, P., L, L.: Deep Learning Based Regression and Multiclass Models for Acute Oral Toxicity Prediction with Automatic Chemical Feature Extraction. Journal of chemical information and modeling 57(11), 2672-2685 (2017). https://doi.org/10.1021/ACS.JCIM.7B00244

[9] Wang, X., Li, Z., Jiang, M., Wang, S., Zhang, S., Wei, Z.: Molecule Property Prediction Based on Spatial Graph Embedding. Journal of Chemical Information and Modeling 59(9), 3817-3828 (2019). https://doi.org/10.1021/ACS.JCIM.9B00410

[10] Gilmer, J., Schoenholz, S.S., Riley, P.F., Vinyals, O., Dahl, G.E.: Neural Message Passing for Quantum Chemistry. 34th International Conference on Machine Learning, ICML 2017 3, 2053-2070 (2017) arXiv:1704.01212v2

[11] Withnall, M., Lindel¨of, E., Engkvist, O., Chen, H.: Building attention and edge message passing neural networks for bioactivity and physical-chemical property prediction. Journal of Cheminformatics 2020 12(1), 1-18 (2020). https://doi.org/10.1186/S13321-019-0407-Y

[12] Velivckovi'c, P., Casanova, A., Li'o, P., Cucurull, G., Romero, A., Bengio, Y.: Graph Attention Networks. 6th International Conference on Learning Representations, ICLR 2018 - Conference Track Proceedings (2017) arXiv:1710.10903. https://doi.org/10.48550/arxiv.1710.10903

[13] Yang, K., Swanson, K., Jin, W., Coley, C., Eiden, P., Gao, H., Guzman-Perez, A., Hopper, T., Kelley, B., Mathea, M., Palmer, A., Settels, V., Jaakkola, T., Jensen, K., Barzilay, R.: Analyzing Learned Molecular Representations for Property Prediction. Journal of Chemical Information and Modeling 59(8), 3370-3388 (2019) arXiv:1904.01561. https://doi.org/10.1021/acs.jcim.9b00237

[14] Weininger, D.: SMILES, a Chemical Language and Information System: 1: Introduction to Methodology and Encoding Rules. Journal of Chemical Information and Computer Sciences 28(1), 31-36 (1988). https://doi.org/10.1021/ci00057a005

[15] Chomsky, N.: Syntactic structures. In: Syntactic Structures. De Gruyter Mouton, (2009)

[16] Asgari, E., Mofrad, M.R.K.: Continuous distributed representation of biological sequences for deep proteomics and genomics. PLoS ONE 10(11) (2015)

[17] Asgari, E., McHardy, A., Mofrad, M.R.K.: Probabilistic variable length segmentation of protein sequences for discriminative motif mining (dimotif) and sequence embedding (protvecx). bioRxiv (2018) https://doi.org/10.1101/345843

[18] Asgari, E.: Life Language Processing: Deep Learning-based Language-agnostic Processing of Proteomics, Genomics/metagenomics, and Human Languages. University of California, Berkeley, (2019)

[19] Elnaggar, A., Heinzinger, M., Dallago, C., Rehawi, G., Wang, Y., Jones, L., Gibbs, T., Feher, T., Angerer, C., Steinegger,M., BHOWMIK, D., Rost, B.: Prottrans: Towards cracking the language of life's code through self-supervised deep learning and high performance computing. BIEEE Trans Pattern Anal Mach Intell, Oct 2022

[20] Schwaller, P., Gaudin, T., L'anyi, D., Bekas, C., Laino, T.: "Found in Translation": predicting outcomes of complex organic chemistry reactions using neural sequence-to-sequence models. Chemical Science 9(28), 6091-6098 (2018) arXiv: 1711.04810. https://doi.org/10.1039/C8SC02339E

[21] Mikolov, T., Sutskever, I., Chen, K., Corrado, G.S., Dean, J.: Distributed representations of words and phrases and their compositionality. Advances in neural information processing systems 26 (2013)

[22] Oztürk, H., Ozkirimli, E., Ozgür, A.: A novel methodology on distributed representations of proteins using their interacting ligands. In: Bioinformatics, vol. 34, pp. 295-303. Oxford University Press, (2018).

[23] Chakravarti, S.K.: Distributed Representation of Chemical Fragments. ACS Omega 3(3), 2825-2836 (2018). https://doi.org/10.1021/acsomega.7b02045

[24] Skinnider, M.A., Greg Stacey, R., Wishart, D.S., Foster, L.J.: Chemical language models enable navigation in sparsely populated chemical space. Nat Mach Intell 3, 759-770 (2021). https://doi.org/10.1038/s42256-021-00368-1

[25] Vaswani, A., Shazeer, N., Parmar, N., Uszkoreit, J., Jones, L., Gomez, A.N., Kaiser, L., Polosukhin, I.: Attention is all you need. In: Advances in Neural Information Processing Systems, vol. 2017-Decem, pp. 5999-6009. Neural information processing systems foundation, (2017). https://arxiv.org/abs/1706.03762v5

[26] Chithrananda, S., Grand, G., Ramsundar, B.: ChemBERTa: Large-Scale Self-Supervised Pretraining for Molecular Property Prediction. http://arxiv.org/abs/2010.09885 (2020)

[27] Irwin, R., Dimitriadis, S., He, J., Bjerrum, E.J.: Chemformer: a pretrained transformer for computational chemistry. Machine Learning: Science and Technology 3(1), 015022 (2022). https://doi.org/10.1088/2632-2153/ac3ffb

[28] Devlin, J., Chang, M.-W., Lee, K., Toutanova, K.: BERT: Pre-trainingof deep bidirectional transformers for language understanding. In: Proceedings of the 2019 Conference of the North American Chapter of the Association for Computational Linguistics: Human Language Technologies, Volume 1 (Long and Short Papers), pp. 4171-4186. Association for Computational Linguistics, Minneapolis, Minnesota (2019). https://doi.org/10.18653/v1/N19-1423. https://aclanthology.org/N19-1423

[29] Dong, L., Yang, N., Wang, W., Wei, F., Liu, X., Wang, Y., Gao, J., Zhou, M., Hon, H.-W.: Unified Language Model Pre-Training for Natural Language Understanding and Generation, pp. 13063-13075. Curran Associates Inc., Red Hook, NY, USA (2019)

[30] Varnek, A., Gaudin, C., Marcou, G., Baskin, I., Pandey, A.K., Tetko, I.V.: Inductive transfer of knowledge: Application of multi-task learning and Feature Net approaches to model tissue-air partition coefficients. Journal of Chemical Information and Modeling 49(1), 133-144 (2009). https://doi.org/10.1021/ci8002914

[31] Vig, J., Belinkov, Y., John, H., Paulson, A.: Analyzing the Structure of Attention in a Transformer Language Model. In: Proceedings of the 2019 ACL Workshop BlackboxNLP: Analyzing and Interpreting Neural Networks for NLP, pp. 63-76 (2019)

[32] Schütz, C., Ho, D.K., Hamed, M.M., Abdelsamie, A.S., Röhrig, T., Herr, C., Kany, A.M., Rox, K., Schmelz, S., Siebenb¨urger, L., Wirth, M., Börger, C., Yahiaoui, S., Bals, R., Scrima, A., Blankenfeldt, W., Horstmann, J.C., Christmann, R., Murgia, X., Koch, M., Berwanger, A., Loretz, B., Hirsch, A.K.H., Hartmann, R.W., Lehr, C.M., Empting, M.: A New PqsR Inverse Agonist Potentiates Tobramycin Efficacy to Eradicate Pseudomonas aeruginosa Biofilms. Advanced Science 8(12) (2021). https://doi.org/10.1002/ADVS.202004369

[33] Schütz, C., Hodzic, A., Hamed, M., Abdelsamie, A.S., Kany, A.M., Bauer, M., Röhrig, T., Schmelz, S., Scrima, A., Blankenfeldt, W., Empting, M.: Divergent synthesis and biological evaluation of 2-(trifluoromethyl)pyridines as virulence-attenuating inverse agonists targeting PqsR. European journal of medicinal chemistry 226 (2021). https://doi.org/10.1016/J.EJMECH.2021.113797

[34] Hamed, M.M., Abdelsamie, A.S., Rox, K., Schütz, C., Kany, A.M., Röhrig, T., Schmelz, S., Blankenfeldt, W., Arce-Rodriguez, A., Borrero-de Acuna, J.M., Jahn, D., Rademacher, J., Ringshausen, F.C., Cramer, N., Tümmler, B., Hirsch, A.K.H., Hartmann, R.W., Empting, M.: Towards Translation of PqsR Inverse Agonists: From In Vitro Efficacy Optimization to In Vivo Proof-of-Principle. Advanced Science, 2204443 (2023). https://doi.org/10.1002/ADVS.202204443

[35] Zender, M., Witzgall, F., Kiefer, A., Kirsch, B., Maurer, C.K., Kany, A.M., Xu, N., Schmelz, S., Börger, C., Blankenfeldt, W., Empting, M.: Flexible Fragment Growing Boosts Potency of Quorum-Sensing Inhibitors against Pseudomonas aeruginosa Virulence. Chemmedchem 15(2), 188 (2020). https://doi.org/10.1002/CMDC.201900621

[36] Ramsundar, B., Eastman, P., Walters, P., Pande, V., Leswing, K., Wu, Z.: Deep Learning for the Life Sciences. O'Reilly Media,(2019).

[37] Sterling, T., Irwin, J.J.: ZINC 15 - Ligand Discovery for Everyone. Journal of Chemical Information and Modeling 55(11), 2324-2337 (2015). https: //doi.org/10.1021/ACS.JCIM.5B00559

[38] Wu, Z., Ramsundar, B., Feinberg, E.N., Gomes, J., Geniesse, C., Pappu, A.S., Leswing, K., Pande, V.: MoleculeNet: A benchmark for molecular machine learning. Chemical Science 9(2), 513-530 (2018), arXiv:1703.00564. https://doi.org/10.1039/c7sc02664a

[39] Wang, C., Cho, K., Gu, J.: Neural Machine Translation with Byte-Level Subwords. AAAI 2020 - 34th AAAI Conference on Artificial Intelligence, 9154-9160 (2019) arXiv: 1909.03341

[40] Wolf, T., Debut, L., Sanh, V., Chaumond, J., Delangue, C., Moi, A., Cistac, P., Rault, T., Louf, R., Funtowicz, M., Davison, J., Shleifer, S., von Platen, P., Ma, C., Jernite, Y., Plu, J., Xu, C., Scao, T.L., Gugger, S., Drame, M., Lhoest, Q., Rush, A.M.: HuggingFace's Transformers: State-of-the-art Natural Language Processing (2019). http://arxiv.org/abs/1910.03771

[41] Paszke, A., Gross, S., Massa, F., Lerer, A., Bradbury, J., Chanan, G., Killeen, T., Lin, Z., Gimelshein, N., Antiga, L., Desmaison, A., Kopf, A., Yang, E., DeVito, Z., Raison, M., Tejani, A., Chilamkurthy, S., Steiner, B., Fang, L., Bai, J., Chintala, S.: Pytorch: An imperative style, high-performance deep learning library. In: Wallach, H., Larochelle, H., Beygelzimer, A., d'Alch'e-Buc, F., Fox, E., Garnett, R. (eds.) Advances in Neural Information Processing Systems 32, pp. 8024-8035. Curran Associates, Inc., (2019). http://papers.neurips.cc/paper/9015-pytorchan-imperative-style-high-performance-deep-learning-library.pdf

[42] Gage, P.: A new algorithm for data compression. C Users J. 12(2), 23-38 (1994)

[43] Beltagy, I., Lo, K., Cohan, A.: SciBERT: A Pretrained Language Model for Scientific Text. EMNLP-IJCNLP 2019 - 2019 Conference on Empirical Methods in Natural Language Processing and 9th International Joint Conference on Natural Language Processing, Proceedings of the Conference, 3615-3620 (2019) arXiv:1903.10676

[44] Howard, J., Ruder, S.: Universal Language Model Fine-tuning for Text Classification. ACL 2018 - 56th Annual Meeting of the Association for Computational Linguistics, Proceedings of the Conference (Long Papers) 1, 328-339 (2018) arXiv: 1801.06146

[45] Bjerrum, E.J.: SMILES Enumeration as Data Augmentation for Neural Network Modeling of Molecules (2017). http://arxiv.org/abs/1703.07076

[46] G., L.: RDKit: Open-source cheminformatics. https://www.rdkit.org

**Claims**

1. A computer-implemented method for training a model (13) for use in estimation of at least one property (16) of a chemical compound and/or for generating or optimizing chemical compounds, the model comprising a language model (13-1), the method comprising the steps of:

   a) Training the language model (13-1) using a large number of chemical compounds in a string representation (10) as training data,
   b) Performing domain adaption of the trained language model (13-1) of step a) for a target domain using target domain-specific training data of the chemical compounds,
   c) Training the domain-adapted trained model (13) of step b) by supervised training for a specific molecular or other property prediction task and/or a specific chemical compound generation or optimization task.

2. A method according to claim 1, **characterized in that** additional training data is generated using data augmentation and is used at least in step b).

3. A method according to claim 2, **characterized in that** the string representations (10) of chemical compounds are reordered leading to multiple string representations (10) of a molecule.

4. A method according to any one of the preceding claims, **characterized in that** the string representation (10) of the chemical compounds is tokenized by using Byte Pair Encoding (BPE).

5. A method according to any one of the preceding claims, **characterized in that** only a prediction part (13-2) of the model (13) is trained during step c) while parameters of the language model (13-1) are kept constant during step c).

6. A method according to any one of claims 1 to 4, **characterized in that** the language model (13-1) and a prediction part (13-2) of the model (13) are trained during step c).

7. A method according to any one of the preceding claims, **characterized in that** hyperparameters of the model (13) and/or an embeddings' type of the language model (13-1) and/or an augmentation size are optimized using the training data of step c) and/or at least one benchmark dataset.

8. A computer-implemented method for controllable generation or optimization of drugs, wherein a model (13) trained according to any one of claims 1 to 7 is used within a learning framework to controllably generate or optimize one or more chemical compounds.

9. A method according to claim 8, **characterized in that** the learning framework implements reinforcement learning.

10. A method for predicting at least one property of one or more chemical compounds and/or for generating or optimizing at least one chemical compound, wherein the method comprises the method according to any one of claims 1 to 9, and wherein the method further comprises selecting a group of chemical compounds with at least one given target property and/or determining at least one molecular or other property of the group and using at least part of the group for domain adaption in step b) and/or supervised training in step c).

11. Computer program having instructions which when executed by a computing device or system cause the computing device or system to perform the method according to any one of claims 1 to 10.

12. A data-processing system comprising means for carrying out the method according to any one of claims 1 to 10.

13. Use of the model (13) trained according to any one of the preceding claims 1 to 10 to predict at least one molecular or other property (16) and/or a drug-target interaction and/or a drug potency and/or a binding to proteins of a chemical compound.

14. Use of the model (13) trained according to any one of the preceding claims 1 to 10 to generate or optimize one or more chemical compounds with at least one specific target property.

15. A trained model (13) obtainable by a training method according to any one of claims 1 to 10.

Fig. 1

Fig. 2

Fig. 3

Fig. 4

Fig. 5

Fig. 6

Europäisches
Patentamt

European
Patent Office

Office européen
des brevets

**EUROPEAN SEARCH REPORT**

Application Number

EP 23 18 9591

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI XINHAO ET AL: "Inductive transfer learning for molecular activity prediction: Next-Gen QSAR Models with MolPMoFiT", JOURNAL OF CHEMINFORMATICS, vol. 12, no. 27, 1 December 2020 (2020-12-01), pages 1-15, XP055877954, DOI: 10.1186/s13321-020-00430-x Retrieved from the Internet: URL:https://jcheminf.biomedcentral.com/track/pdf/10.1186/s13321-020-00430-x.pdf> * the whole document * | 1-15 | INV. G16C20/70 G16C20/30 G16C20/50 G06N3/045 ADD. G06N3/08 |
| A | INGOO LEE ET AL: "Infusing Linguistic Knowledge of SMILES into Chemical Language Models", ARXIV.ORG, CORNELL UNIVERSITY LIBRARY, 201 OLIN LIBRARY CORNELL UNIVERSITY ITHACA, NY 14853, 20 April 2022 (2022-04-20), XP091211456, * the whole document * | 1-15 | |
| A | WO 2022/162094 A1 (SANOFI SA [FR]; ECOLE NORMALE SUPERIEURE [FR] ET AL.) 4 August 2022 (2022-08-04) * the whole document * | 1-15 | TECHNICAL FIELDS SEARCHED (IPC) G16C G06N |

-/--

The present search report has been drawn up for all claims

| Place of search | Date of completion of the search | Examiner |
|---|---|---|
| The Hague | 11 January 2024 | Denoual, Matthieu |

Europäisches
Patentamt

European
Patent Office

Office européen
des brevets

# EUROPEAN SEARCH REPORT

Application Number

**EP 23 18 9591**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Kallergis Georgios ET AL: "Domain adaptable language modeling of chemical compounds identifies potent pathoblockers for Pseudomonas aeruginosa.", , 18 December 2023 (2023-12-18), XP093117942, DOI: 10.26434/chemrxiv-2023-cpkfk Retrieved from the Internet: URL:https://chemrxiv.org/engage/api-gateway/chemrxiv/assets/orp/resource/item/657cb14de9ebbb4db9fa0e13/original/domain-adaptable-language-modeling-of-chemical-compounds-identifies-potent-pathoblockers-for-pseudomonas-aeruginosa.pdf [retrieved on 2024-01-10] * the whole document * ----- | | |
| | | | **TECHNICAL FIELDS SEARCHED (IPC)** |

The present search report has been drawn up for all claims

| Place of search | Date of completion of the search | Examiner |
|---|---|---|
| The Hague | 11 January 2024 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 9591

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2024

| Patent document cited in search report | Publication date | Patent family member(s) | Publication date |
|---|---|---|---|
| WO 2022162094 A1 | 04-08-2022 | EP 4252240 A1<br>WO 2022162094 A1 | 04-10-2023<br>04-08-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PAUL, S.M.** ; **MYTELKA, D.S.** ; **DUNWIDDIE, C.T.** ; **PERSINGER, C.C.** ; **MUNOS, B.H.** ; **LINDBORG, S.R.** ; **SCHACHT, A.L.** *How to improve RD productivity: The pharmaceutical industry's grand challenge*, 2010, vol. 9 (3), 203-214, https://doi.org/10.1038/nrd3078
- **HINGORANI, A.D.** ; **KUAN, V.** ; **FINAN, C.** ; **KRUGER, F.A.** ; **GAULTON, A.** ; **CHOPADE, S.** ; **SOFAT, R.** ; **MACALLISTER, R.J.** ; **OVERINGTON, J.P.** ; **HEMINGWAY, H.** Improving the odds of drug development success through human genomics: modelling study. *Scientific Reports*, 2019, vol. 9 (1), 18911, https://doi.org/10.1038/s41598-019-54849-w
- **MA, J.** ; **SHERIDAN, R.P.** ; **LIAW, A.** ; **DAHL, G.E.** ; **SVETNIK, V.** Deep neural nets as a method for quantitative structure-activity relationships. *Journal of Chemical Information and Modeling*, 2015, vol. 55 (2), 263-274, https:// doi.org/10.1021/ci500747n
- **CHEN, H.** ; **ENGKVIST, O.** ; **WANG, Y.** ; **OLIVE-CRONA, M.** ; **BLASCHKE, T.** The rise of deep learning in drug discovery. Elsevier Ltd, 2018
- **ZHANG, Q.Y.** ; **AIRES-DE-SOUSA, J.** Random forest prediction of mutagenicity from empirical physicochemical descriptors. *Journal of Chemical Information and Modeling*, 2007, vol. 47 (1), 1-8, https://doi.org/10.1021/ci050520j
- **ZERNOV, V.V.** ; **BALAKIN, K.V.** ; **IVASCHENKO, A.A.** ; **SAVCHUK, N.P.** ; **PLETNEV, I.V.** Drug Discovery Using Support Vector Machines. The Case Studies of Drug-likeness, Agrochemical-likeness, and Enzyme Inhibition Predictions. *Journal of Chemical Information and Computer Sciences*, 2003, vol. 43 (6), 2048-2056, https://doi.org/10.1021/-ci0340916
- **D DUVENAUD** ; **D MACLAURIN, J.A.-I**. Convolutional networks on graphs for learning molecular fingerprints. *Adv Neural Inf Process Syst*, 2015, vol. 2015, 2224-2232
- **Y, X.** ; **J, P.** ; **L, L.** Deep Learning Based Regression and Multiclass Models for Acute Oral Toxicity Prediction with Automatic Chemical Feature Extraction. *Journal of chemical information and modeling*, 2017, vol. 57 (11), 2672-2685, https://doi.org/10.1021/ACS.JCIM.7B00244

- **WANG, X.** ; **LI, Z.** ; **JIANG, M.** ; **WANG, S.** ; **ZHANG, S.** ; **WEI, Z.** Molecule Property Prediction Based on Spatial Graph Embedding. *Journal of Chemical Information and Modeling*, 2019, vol. 59 (9), 3817-3828, https://doi.org/10.1021/ACS.JCIM.9B00410
- **GILMER, J.** ; **SCHOENHOLZ, S.S.** ; **RILEY, P.F.** ; **VINYALS, O.** ; **DAHL, G.E.** Neural Message Passing for Quantum Chemistry. *34th International Conference on Machine Learning, ICML*, 2017, vol. 3, 2053-2070
- **WITHNALL, M.** ; **LINDEL¨OF, E.** ; **ENGKVIST, O.** ; **CHEN, H.** Building attention and edge message passing neural networks for bioactivity and physical-chemical property prediction. *Journal of Cheminformatics*, 2020, vol. 12 (1), 1-18, https://doi.org/10.1186/S13321-019-0407-Y
- **VELIVCKOVI'C, P.** ; **CASANOVA, A.** ; **LI'O, P.** ; **CUCURULL, G.** ; **ROMERO, A.** ; **BENGIO, Y.** Graph Attention Networks. *6th International Conference on Learning Representations, ICLR 2018 - Conference Track Proceedings*, 2017, https://doi.org/10.48550/arxiv.1710.10903
- **YANG, K.** ; **SWANSON, K.** ; **JIN, W.** ; **COLEY, C.** ; **EIDEN, P.** ; **GAO, H.** ; **GUZMAN-PEREZ, A.** ; **HOPPER, T.** ; **KELLEY, B.** ; **MATHEA, M.** Analyzing Learned Molecular Representations for Property Prediction. *Journal of Chemical Information and Modeling*, 2019, vol. 59 (8), 3370-3388, https://doi.org/10.1021/acs.jcim.9b00237
- **WEININGER, D.** SMILES, a Chemical Language and Information System: 1: Introduction to Methodology and Encoding Rules. *Journal of Chemical Information and Computer Sciences*, 1988, vol. 28 (1), 31-36, https://doi.org/10.1021/ci00057a005
- **CHOMSKY, N.** Syntactic structures. *Syntactic Structures. De Gruyter Mouton*, 2009
- **ASGARI, E.** ; **MOFRAD, M.R.K.** Continuous distributed representation of biological sequences for deep proteomics and genomics. *PLoS ONE*, vol. 10 (11), 2015
- **ASGARI, E.** ; **MCHARDY, A.** ; **MOFRAD, M.R.K.** Probabilistic variable length segmentation of protein sequences for discriminative motif mining (dimotif) and sequence embedding (protvecx). *bioRxiv*, 2018, https://doi.org/10.1101/345843

- Life Language Processing: Deep Learning-based Language-agnostic Processing of Proteomics. **AS-GARI, E.** Genomics/metagenomics, and Human Languages. University of California, 2019
- **ELNAGGAR, A.** ; **HEINZINGER, M.** ; **DALLAGO, C.** ; **REHAWI, G.** ; **WANG, Y.** ; **JONES, L.** ; **GIBBS, T.** ; **FEHER, T.** ; **ANGERER, C.** ; **STEINEGGER,M.** Prottrans: Towards cracking the language of life's code through self-supervised deep learning and high performance computing. *BIEEE Trans Pattern Anal Mach Intell*, October 2022
- **SCHWALLER, P.** ; **GAUDIN, T.** ; **L'ANYI, D.** ; **BEKAS, C.** ; **LAINO, T.** Found in Translation. *Chemical Science*, 2018, vol. 9 (28), 6091-6098, https://doi.org/10.1039/C8SC02339E
- **MIKOLOV, T.** ; **SUTSKEVER, I.** ; **CHEN, K.** ; **CORRADO, G.S.** ; **DEAN, J.** Distributed representations of words and phrases and their compositionality. *Advances in neural information processing systems*, 2013, vol. 26
- A novel methodology on distributed representations of proteins using their interacting ligands. **OZTÜRK, H.** ; **OZKIRIMLI, E.** ; **OZGÜR, A.** Bioinformatics. Oxford University Press, 2018, vol. 34, 295-303
- **CHAKRAVARTI, S.K.** Distributed Representation of Chemical Fragments. *ACS Omega*, 2018, vol. 3 (3), 2825-2836, https://doi.org/10.1021/acsomega.7b02045
- **SKINNIDER, M.A.** ; **GREG STACEY, R.** ; **WISHART, D.S.** ; **FOSTER, L.J.** Chemical language models enable navigation in sparsely populated chemical space. *Nat Mach Intell*, 2021, vol. 3, 759-770, https://doi.org/10.1038/s42256-021-00368-1
- **VASWANI, A.** ; **SHAZEER, N.** ; **PARMAR, N.** ; **USZKOREIT, J.** ; **JONES, L.** ; **GOMEZ, A.N.** ; **KAISER, L.** ; **POLOSUKHIN, I.** Attention is all you need. *Advances in Neural Information Processing Systems*, December 2017, vol. 2017, 5999-6009, https://arxiv.org/abs/1706.03762v5
- **CHITHRANANDA, S.** ; **GRAND, G.** ; **RAMSUNDAR, B.** *ChemBERTa: Large-Scale Self-Supervised Pretraining for Molecular Property Prediction*, 2020, http://arxiv.org/abs/2010.09885
- **IRWIN, R.** ; **DIMITRIADIS, S.** ; **HE, J.** ; **BJERRUM, E.J.** Chemformer: a pretrained transformer for computational chemistry. *Machine Learning: Science and Technology*, 2022, vol. 3 (1), 015022, https://doi.org/10.1088/2632-2153/ac3ffb
- BERT: Pre-trainingof deep bidirectional transformers for language understanding. **DEVLIN, J.** ; **CHANG, M.-W.** ; **LEE, K.** ; **TOUTANOVA, K.** Proceedings of the 2019 Conference of the North American Chapter of the Association for Computational Linguistics: Human Language Technologies. Association for Computational Linguistics, 2019, vol. 1, 4171-4186
- **DONG, L.** ; **YANG, N.** ; **WANG, W.** ; **WEI, F.** ; **LIU, X.** ; **WANG, Y.** ; **GAO, J.** ; **ZHOU, M.** ; **HON, H.-W.** Unified Language Model Pre-Training for Natural Language Understanding and Generation. Curran Associates Inc., 2019, 13063-13075
- **VARNEK, A.** ; **GAUDIN, C.** ; **MARCOU, G.** ; **BASKIN, I.** ; **PANDEY, A.K.** ; **TETKO, I.V.** Inductive transfer of knowledge: Application of multi-task learning and Feature Net approaches to model tissue-air partition coefficients. *Journal of Chemical Information and Modeling*, 2009, vol. 49 (1), 133-144, https://doi.org/10.1021/ci8002914
- **VIG, J.** ; **BELINKOV, Y.** ; **JOHN, H.** ; **PAULSON, A.** Analyzing the Structure of Attention in a Transformer Language Model. *Proceedings of the 2019 ACL Workshop BlackboxNLP: Analyzing and Interpreting Neural Networks for NLP*, 2019, 63-76
- **SCHÜTZ, C.** ; **HO, D.K.** ; **HAMED, M.M.** ; **ABDEL-SAMIE, A.S.** ; **RÖHRIG, T.** ; **HERR, C.** ; **KANY, A.M.** ; **ROX, K.** ; **SCHMELZ, S.** ; **SIEBENB¨URGER, L.** A New PqsR Inverse Agonist Potentiates Tobramycin Efficacy to Eradicate Pseudomonas aeruginosa Biofilms. *Advanced Science*, 2021, vol. 8 (12), https://doi.org/10.1002/ADVS.202004369
- **SCHÜTZ, C.** ; **HODZIC, A.** ; **HAMED, M.** ; **ABDEL-SAMIE, A.S.** ; **KANY, A.M.** ; **BAUER, M.** ; **RÖHRIG, T.** ; **SCHMELZ, S.** ; **SCRIMA, A.** ; **BLANKENFELDT, W.** Divergent synthesis and biological evaluation of 2-(trifluoromethyl)pyridines as virulence-attenuating inverse agonists targeting PqsR. *European journal of medicinal chemistry*, 2021, vol. 226, https://doi.org/10.1016/J.EJMECH.2021.113797
- **HAMED, M.M.** ; **ABDELSAMIE, A.S.** ; **ROX, K.** ; **SCHÜTZ, C.** ; **KANY, A.M.** ; **RÖHRIG, T.** ; **SCHMELZ, S.** ; **BLANKENFELDT, W.** ; **ARCE-RODRIGUEZ, A.** ; **BORRERO-DE ACUNA, J.M.** Towards Translation of PqsR Inverse Agonists: From In Vitro Efficacy Optimization to In Vivo Proof-of-Principle. *Advanced Science*, 2023, 2204443, https://doi.org/10.1002/ADVS.202204443
- **ZENDER, M.** ; **WITZGALL, F.** ; **KIEFER, A.** ; **KIRSCH, B.** ; **MAURER, C.K.** ; **KANY, A.M.** ; **XU, N.** ; **SCHMELZ, S.** ; **BÖRGER, C.** ; **BLANKEN-FELDT, W.** Flexible Fragment Growing Boosts Potency of Quorum-Sensing Inhibitors against Pseudomonas aeruginosa Virulence. *Chemmedchem*, 2020, vol. 15 (2), 188, https://doi.org/10.1002/CMDC.201900621
- **RAMSUNDAR, B.** ; **EASTMAN, P.** ; **WALTERS, P.** ; **PANDE, V.** ; **LESWING, K.** ; **WU, Z.** Deep Learning for the Life Sciences. O'Reilly Media, 2019
- **STERLING, T.** ; **IRWIN, J.J.** ZINC 15 - Ligand Discovery for Everyone. *Journal of Chemical Information and Modeling*, 2015, vol. 55 (11), 2324-2337, https://doi.org/10.1021/ACS.JCIM.5B00559

- **WU, Z.** ; **RAMSUNDAR, B.** ; **FEINBERG, E.N.** ; **GOMES, J.** ; **GENIESSE, C.** ; **PAPPU, A.S.** ; **LESWING, K.** ; **PANDE, V.** MoleculeNet: A benchmark for molecular machine learning. *Chemical Science*, 2018, vol. 9 (2), 513-530, https://doi.org/10.1039/c7sc02664a
- **WANG, C.** ; **CHO, K.** ; **GU, J.** Neural Machine Translation with Byte-Level Subwords. *AAAI 2020 - 34th AAAI Conference on Artificial Intelligence*, 2019, 9154-9160
- **WOLF, T.** ; **DEBUT, L.** ; **SANH, V.** ; **CHAUMOND, J.** ; **DELANGUE, C.** ; **MOI, A.** ; **CISTAC, P.** ; **RAULT, T.** ; **LOUF, R.** ; **FUNTOWICZ, M.** *HuggingFace's Transformers: State-of-the-art Natural Language Processing*, 2019, http://arxiv.org/abs/1910.03771
- Pytorch: An imperative style, high-performance deep learning library. **PASZKE, A.** ; **GROSS, S.** ; **MASSA, F.** ; **LERER, A.** ; **BRADBURY, J.** ; **CHANAN, G.** ; **KILLEEN, T.** ; **LIN, Z.** ; **GIMELSHEIN, N.** ; **ANTIGA, L.** Advances in Neural Information Processing Systems. Curran Associates, Inc., 2019, vol. 32, 8024-8035
- **GAGE, P.** A new algorithm for data compression. *C Users J.*, 1994, vol. 12 (2), 23-38
- **BELTAGY, I.** ; **LO, K.** ; **COHAN, A.** SciBERT: A Pretrained Language Model for Scientific Text. EMNLP-IJCNLP 2019 - 2019 Conference on Empirical Methods in Natural Language Processing and 9th International Joint Conference on Natural Language Processing. *Proceedings of the Conference*, 2019, 3615-3620
- **HOWARD, J.** ; **RUDER, S.** Universal Language Model Fine-tuning for Text Classification. *ACL 2018 - 56th Annual Meeting of the Association for Computational Linguistics, Proceedings of the Conference (Long Papers)*, 2018, vol. 1, 328-339
- **BJERRUM, E.J.** *SMILES Enumeration as Data Augmentation for Neural Network Modeling of Molecules*, 2017, http://arxiv.org/abs/1703.07076
- **G., L.** *RDKit: Open-source cheminformatics*, https://www.rdkit.org